(19) Europäisches Patentamt
European Patent Office
Office européen des brevets

(11) Veröffentlichungsnummer: **0 032 395**
**B1**

(12) # EUROPÄISCHE PATENTSCHRIFT

(45) Veröffentlichungstag der Patentschrift: **23.03.83**

(51) Int. Cl.³: **C 07 D 239/49**

(21) Anmeldenummer: **81100161.9**

(22) Anmeldetag: **03.11.78**

(60) Veröffentlichungsnummer der früheren Anmeldung nach Art. 76 EPÜ:

(54) **Benzylpyrimidine.**

(30) Priorität: **10.11.77 LU 78487**
**19.09.78 CH 9776/78**

(43) Veröffentlichungstag der Anmeldung: **22.07.81 Patentblatt 81/29**

(45) Bekanntmachung des Hinweises auf die Patenterteilung: **23.03.83 Patentblatt 83/12**

(84) Benannte Vertragsstaaten: **BE CH DE FR LU NL SE**

(56) Entgegenhaltungen:
**DE-A-2 165 362**
**DE-A-2 258 238**
**DE-A-2 558 150**

(73) Patentinhaber: **F. HOFFMANN-LA ROCHE & CO. Aktiengesellschaft, CH-4002 Basel (CH)**

(72) Erfinder: **Kompis, Ivan, Dr., Lettenhofstrasse 6, CH-4104 Oberwil (CH)**
Erfinder: **Wick, Alexander Eduard, Dr., Rue du Buisson Richard 10, F-78600 Le Mesnil-le-Roy (FR)**

(74) Vertreter: **Lederer, Franz, Dr. et al, Patentanwälte Dr. Franz Lederer Reiner F. Meyer Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

## Benzylpyrimidine

Die vorliegende Erfindung betrifft neue Benzylpyrimidine der allgemeinen Formel

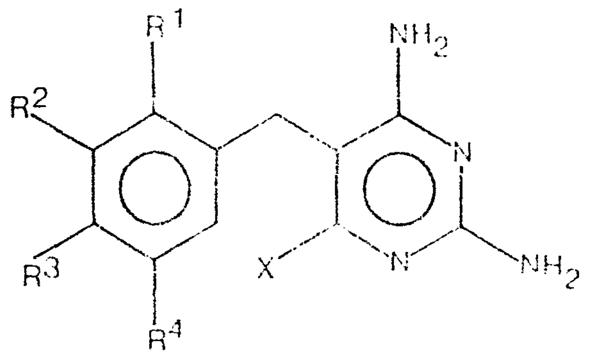

III

worin

$R^1$ Wasserstoff oder $C_{1-6}$-Alkyl; $R^2$ Wasserstoff und $R^3$ und $R^4$ $C_{1-6}$-Alkylthio oder
$R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff; $R^2$ $C_{1-6}$-Alkoxy und $R^3$ und $R^4$ $C_{1-6}$-Alkylthio oder
$R^1$ Wasserstoff; $R^2$ und $R^4$ $C_{1-6}$-Alkoxy und $R^3$ $C_{1-6}$-Alkylthio, Carboxy-$C_{1-6}$-alkylthio oder Cyano-$C_{1-6}$-alkylthio darstellen und
X Halogen ist.

Beispiele für $C_{1-6}$-Alkylreste sind Methyl, Äthyl, Propyl, Isopropyl, Butyl, sek.-Butyl, Isobutyl, tert.-Butyl, Pentyl und Hexyl, wobei Methyl und Äthyl bevorzugt sind.

Eine besonders bevorzugte Verbindung der Formel III ist 2,6-Diamino-4-chlor-5-(3,5-dimethoxy-4-methylthiobenzyl)-pyrimidin.

Die Herstellung der Verbindungen III kann in an sich bekannter Weise aus entsprechenden 2,6-Diamino-4-hydroxy-5-benzylpyrimidinen der Formel XII (Reaktionsschema I) durch Umsetzung mit einem Phosphoroxyhalogenid erfolgen. Die Verbindungen XII können aus entsprechend substituierten Aldehyden der Formel VIII in Analogie zu bekannten Verfahren, wie im Reaktionsschema I dargestellt, hergestellt werden. Bei den Aldehyden der Formel VIII (in denen die Substituenten $R^1-R^4$ die gleichen Bedeutungen haben wie in den Verbindungen der Formel III) handelt es sich um neue Verbindungen (mit Ausnahme des 3,4-Bis-(methylthio)-benzaldehyds), deren Herstellung aus bekannten Verbindungen in den Reaktionsschemata II—V zusammengefaßt ist.

Durch reduktive Eliminierung des Substituenten X, der vorzugsweise ein Chlor- oder Bromatom ist, können die Verbindungen der Formel III in pharmakologisch wirksame 2,4-Diamino-5-benzylpyrimidine der Formel

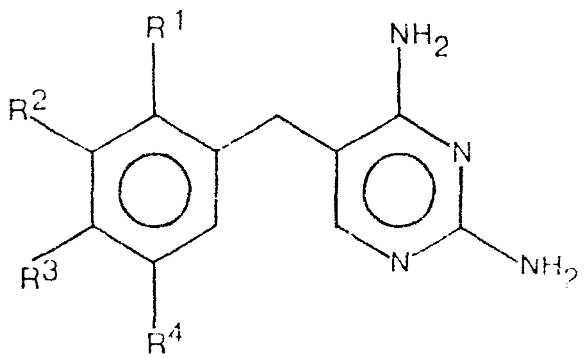

I

worin $R^1-R^4$ die gleichen Bedeutungen wie in Formel III haben, überführt werden.

## Reaktionsschema I

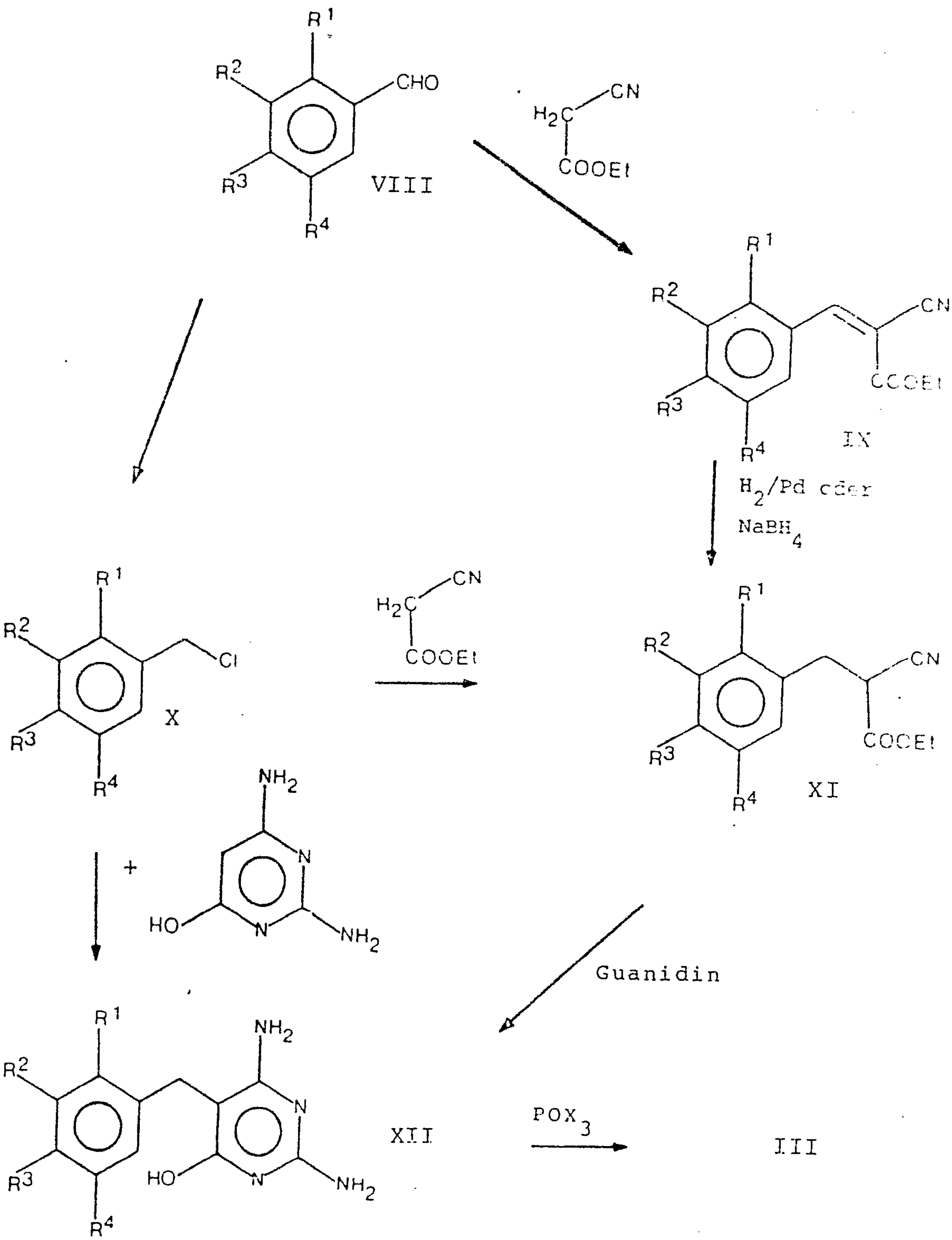


VIII IX X XI XII III

Reaktionsschema II

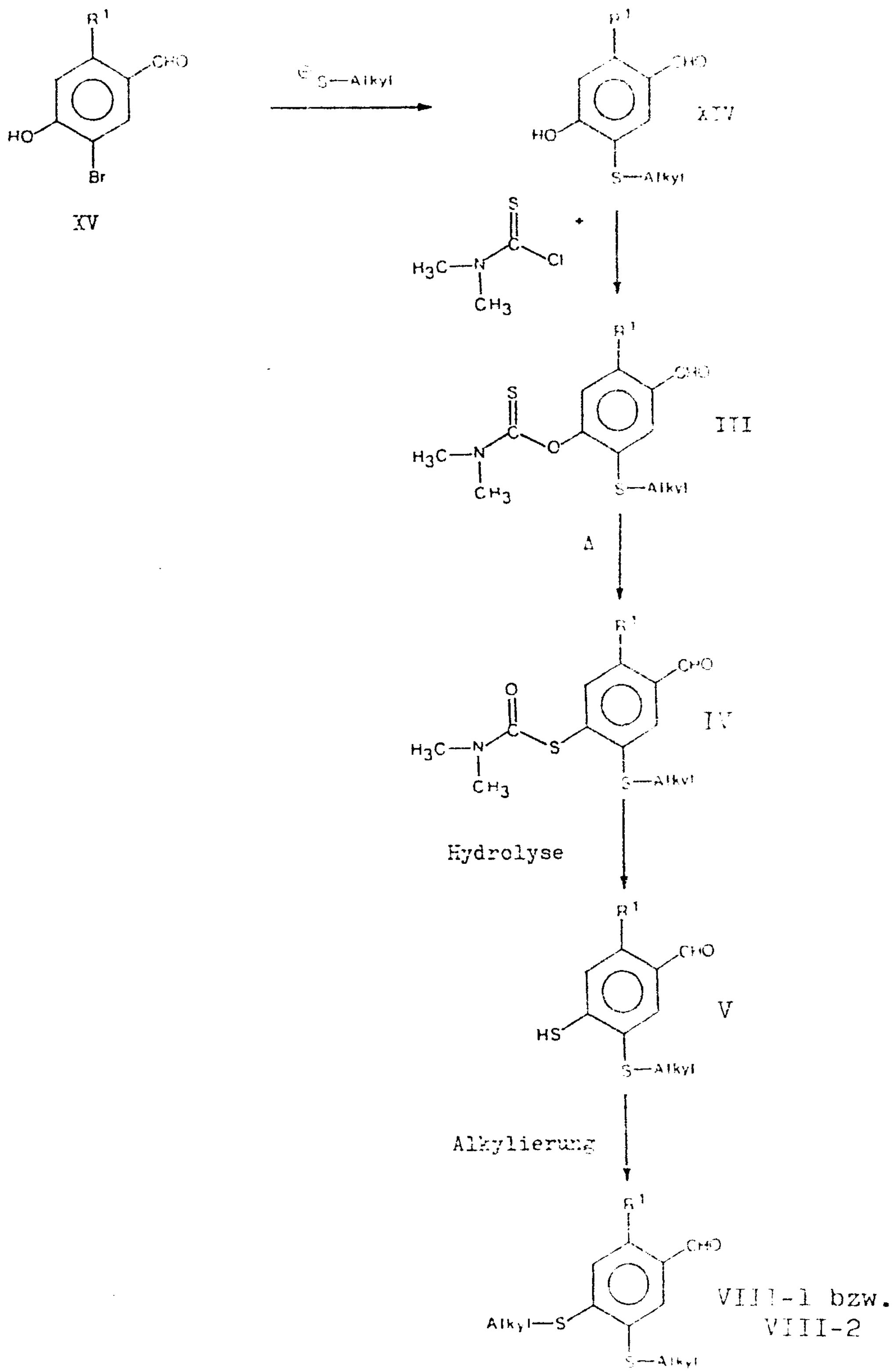


XV, XIV, III, IV, V, VIII-1 bzw. VIII-2

Reaktionsschema III

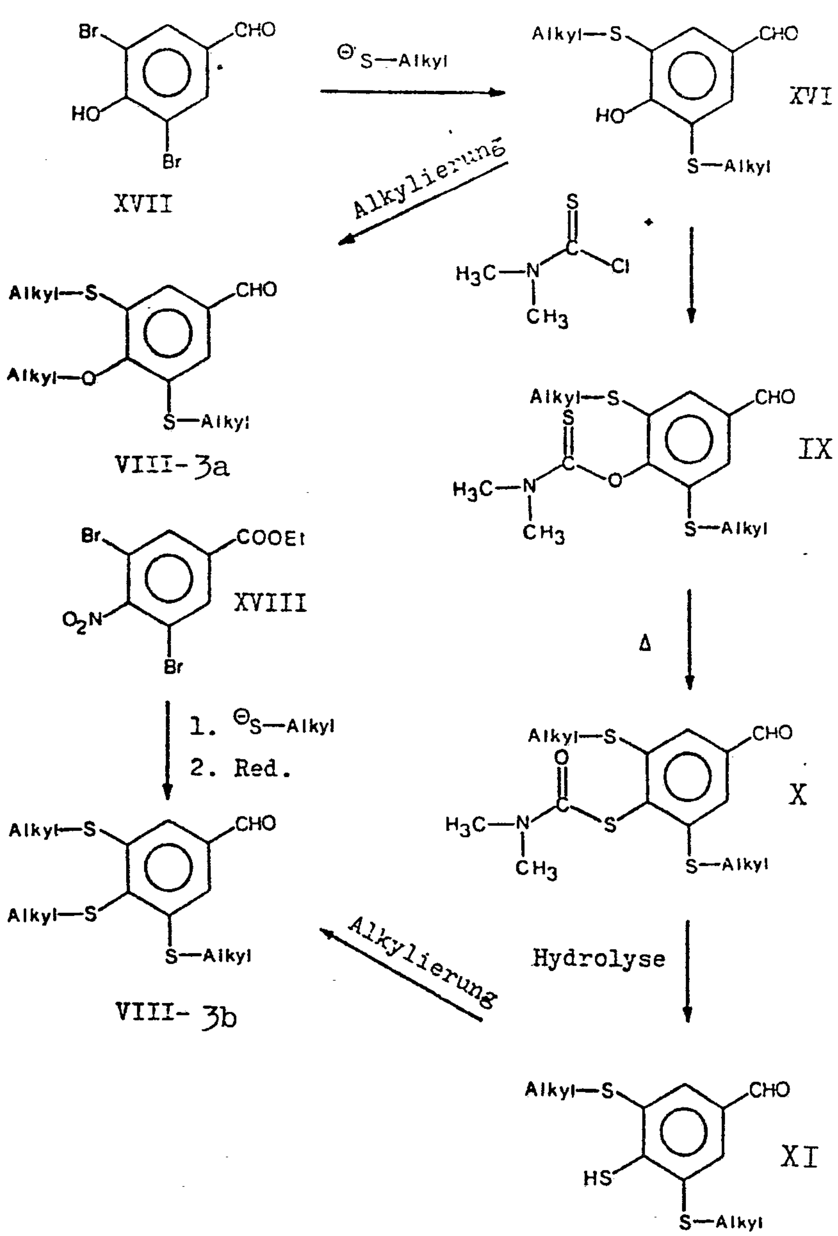


XVII, XVI, VIII-3a, IX, XVIII, X, VIII-3b, XI

Reaktionsschema IV

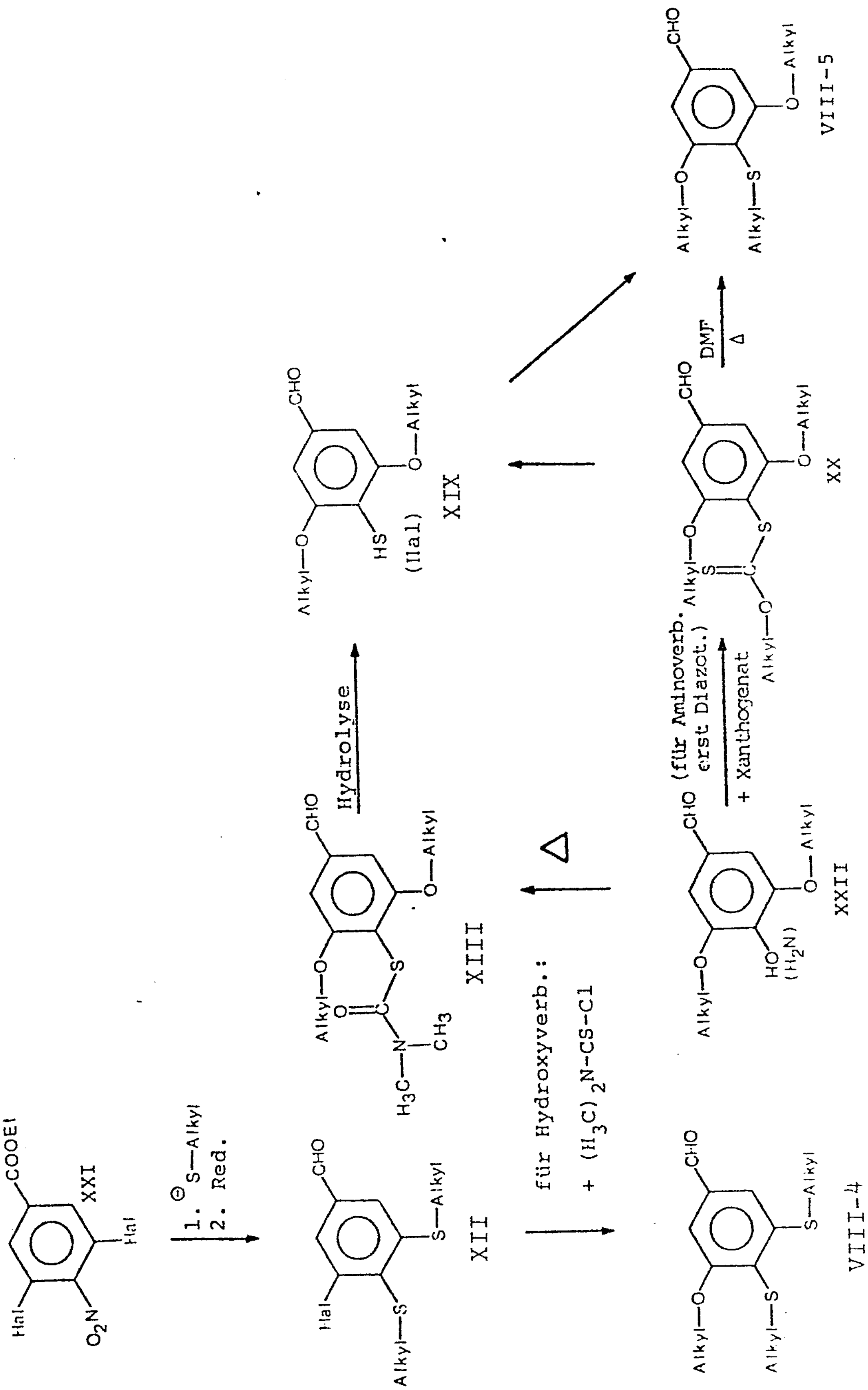

Reaktionsschema V

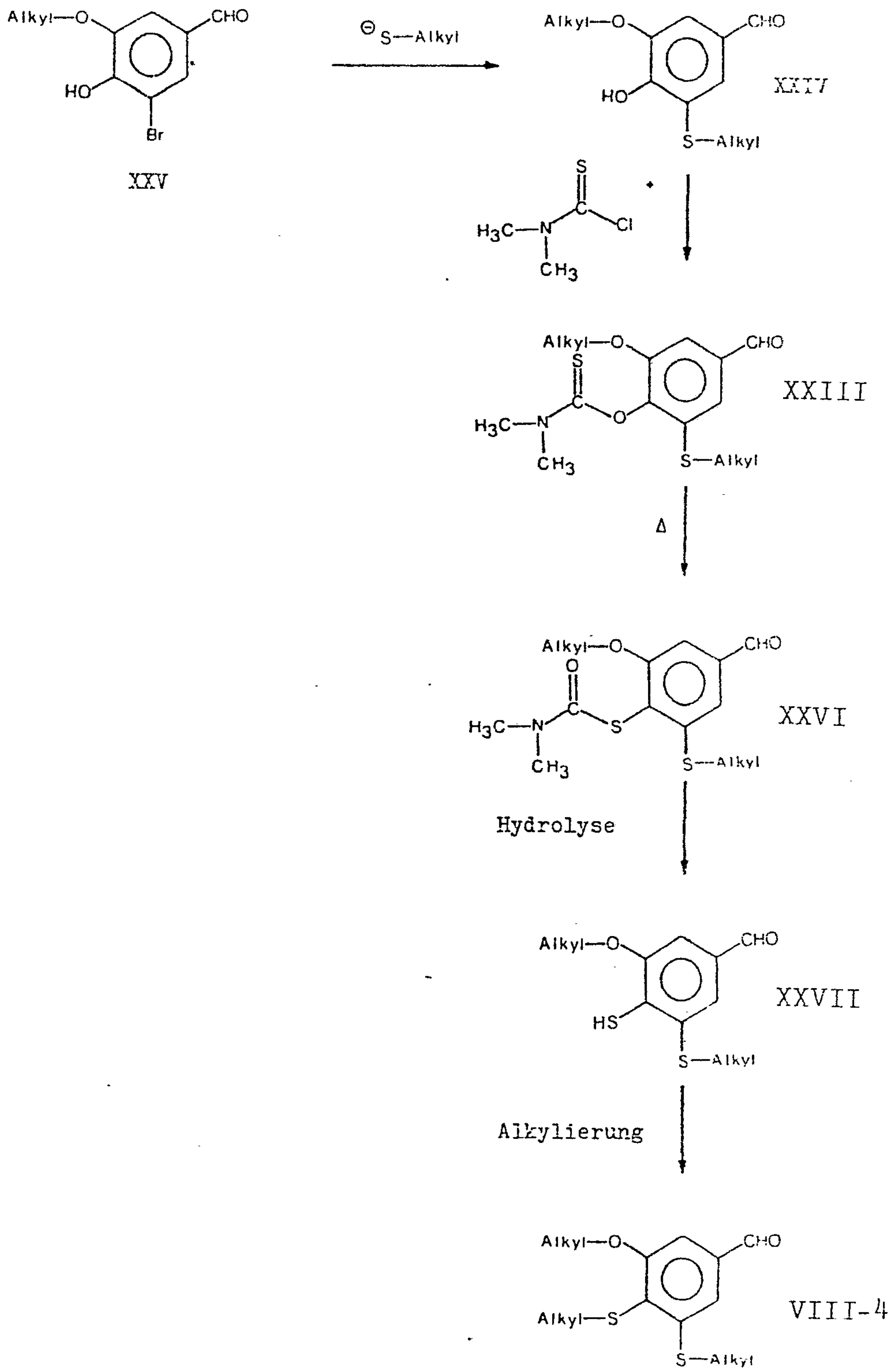


XXV

XXIV

XXIII

XXVI

XXVII

VIII-4

Die reduktive Eliminierung des Substituenten X kann in an sich bekannter Weise (siehe z. B. DE-A-2 165 362, DE-A-2 258 150, DE-A-2 558 150) durch Behandlung einer Verbindung III mit Reduktionsmitteln wie Zink in Eisessig, amalgamiertem Zink in Natronlauge oder durch katalytische Hydrierung, beispielsweise in Gegenwart eines Edelmetallkatalysators, wie Palladium, in einem inerten Lösungsmittel, wie Äthanol, erfolgen.

Die Verbindungen der Formel I selbst sind Gegenstand des europäischen Patents Nr. 0 003 212.

## Beispiel

Ein Gemisch aus 10,6 g 3,5-Dimethoxy-4-methylthiobenzaldehyd, 6,2 g Cyanessigsäureäthylester und 2 Tropfen Piperidin wurde im offenen Kolben unter Rühren 90 Minuten auf 120° C erwärmt, dann abgekühlt und aus Äther/n-Heptan umkristallisiert. Die Ausbeute an α-Cyano-3,5-dimethoxy-4-methylthiozimtsäureäthylester, Smp. 98—100° C (aus Äthanol), betrug 12,0 g (80%).

Zu einer Suspension von 6,0 g α-Cyano-3,5-dimethoxy-4-methylthio-zimtsäureäthylester in 130 ml Äthanol und 2 Tropfen 1N Natronlauge wurden unter Rühren 0,23 g Natriumborhydrid gegeben, wobei eine Lösung entstand. Nach 30 Minuten wurde unter Wasserstrahlvakuum bei 40° C das Äthanol abgedampft, der Rückstand in Äther aufgenommen, die Lösung mit Wasser gewaschen und durch Kieselgel filtriert. Es wurden 5,0 g (79,1%) α-Cyano-3,5-dimethoxy-4-methylthio-hydrozimtsäureäthylester als farbloses Öl erhalten.

In einem anderen Versuch wurde eine Lösung von 3 g α-Cyano-3,5-dimethoxy-4-methylthio-zimtsäureäthylester in 250 ml Methanol und 500 mg 5% Pd/C 24 Stunden unter Normaldruck und bei Raumtemperatur hydriert. Nach üblicher Aufarbeitung wurden 3,0 g ( ~ 100%) α-Cyano-3,5-dimethoxy-4-methylthio-hydrozimtsäureäthylester isoliert.

Zu einer Lösung von 0,7 g Natrium in 60 ml abs. Äthanol wurden 2,7 g Guanidinhydrochlorid gegeben. Nach 30 Minuten wurde die Suspension filtriert und zum Filtrat 4,5 g α-Cyano-3,5-dimethoxy-4-methylthio-hydrozimtsäureäthylester gegeben. Das Reaktionsgemisch wurde 18 Stunden unter Rückfluß gekocht, zur Trockne eingedampft, der Rückstand mit Wasser aufgeschlemmt und dreimal mit 200 ml Essigester extrahiert. Der Extrakt wurde eingedampft und der Rückstand aus Methanol kristallisiert. Es wurden 1,8 g (38,5%) 2,6-Diamino-5-(3,5-dimethoxy-4-methylthiobenzyl)-4-pyrimidinol, Smp. 213—215° C, erhalten.

Zu einer Suspension von 0,96 g 2,6-Diamino-5-(3,5-dimethoxy-4-methylthio-benzyl)-4-pyrimidinol in 7,6 g Phosphoroxychlorid wurden unter Rühren tropfenweise 0,73 g Dimethylanilin gegeben. Die Mischung wurde 4 Stunden unter Rühren gekocht und ungefähr die Hälfte des überschüssigen Phosphoroxychlorids wurde unter vermindertem Druck abdestilliert. Zum Rest wurden ca. 10 g Eis gegeben. Die Suspension wurde dann bei Raumtemperatur 36 Stunden stehengelassen und mit konz. Ammoniaklösung auf pH 10 eingestellt. Die entstandene Suspension wurde durch eine Wasserdampfdestillation von Dimethylanilin befreit. Nach dem Erkalten der wäßrigen Suspension wurde das feste Rohprodukt abgenutscht, in Benzol/Methanol (4 : 1, v/v) gelöst und durch Kieselgel filtriert. Nach Abdampfen des Lösungsmittels wurde das erhaltene 2,6-Diamino-4-chlor-5-(3,5-dimethoxy-4-methylthiobenzyl)-pyrimidin aus Methanol umkristallisiert; Smp. 218—221° C.

Zu einer Lösung von 167,7 mg 2,6-Diamino-4-chlor-5-(3,5-dimethoxy-4-methylthiobenzyl)-pyrimidin in 1,6 ml Eisessig und 10 mg Quecksilber(II)-chlorid in 0,2 ml Wasser wurden 150 mg Zinkpulver gegeben. Das Gemisch wurde über Nacht unter Rühren und Rückfluß gekocht. Die erhaltene Lösung wurde heiß filtriert, wobei nicht umgesetztes Zink mit 0,5 ml Eisessig nachgewaschen wurde. Das Filtrat wurde mit 2 ml Wasser verdünnt und unter Kühlung mit konz. Ammoniaklösung alkalisch gestellt. Die Suspension wurde dreimal mit 5 ml Essigester extrahiert, der Extrakt getrocknet und eingeengt und der Rückstand aus Methanol umkristallisiert. Es wurden 148 mg 2,4-Diamino-5-(3,5-dimethoxy-4-methylthio-benzyl)-pyrimidin, Smp. 231—232° C, erhalten.

**Patentansprüche**

1. Benzylpyrimidine der allgemeinen Formel

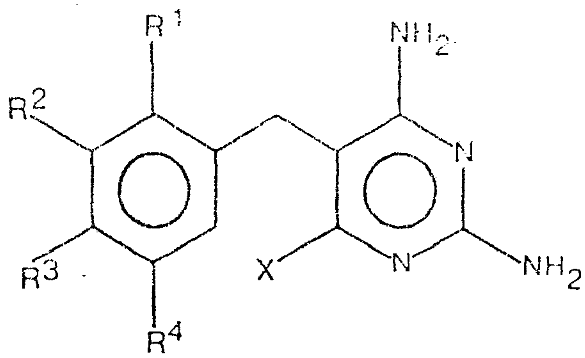

III

worin
$R^1$ Wasserstoff oder $C_{1-6}$-Alkyl; $R^2$ Wasserstoff und $R^3$ und $R^4$ $C_{1-6}$-Alkylthio oder
$R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff; $R^2$ $C_{1-6}$-Alkoxy und $R^3$ und $R^4$ $C_{1-6}$-Alkylthio oder
$R^1$ Wasserstoff; $R^2$ und $R^4$ $C_{1-6}$-Alkoxy und $R^3$ $C_{1-6}$-Alkylthio, Carboxy-$C_{1-6}$-alkylthio oder Cyano-$C_{1-6}$-alkylthio darstellen und
X Halogen ist.

2. Benzylpyrimidine der allgemeinen Formel

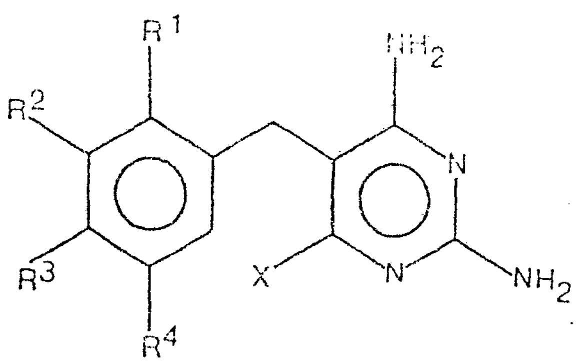

III

worin
$R^1$ Wasserstoff oder $C_{1-6}$-Alkyl; $R^2$ Wasserstoff und $R^3$ und $R^4$ $C_{1-6}$-Alkylthio oder
$R^1$ Wasserstoff; $R^2$ und $R^4$ $C_{1-6}$-Alkylthio und $R^3$ $C_{1-6}$-Alkoxy, $C_{1-6}$-Alkylthio oder Pyrrolo oder
$R^1$ Wasserstoff; $R^2$ $C_{1-6}$-Alkoxy und $R^3$ und $R^4$ $C_{1-6}$-Alkylthio oder
$R^1$ Wasserstoff; $R^2$ und $R^4$ $C_{1-6}$-Alkoxy und $R^3$ $C_{1-6}$-Alkylthio oder Carboxy-$C_{1-6}$-alkylthio darstellen und
X Halogen ist.

3. 2,6-Diamino-4-chlor-5-(3,5-dimethoxy-4-methylthiobenzyl)-pyrimidin.

**Claims**

1. Benzylpyrimidines of the general formula

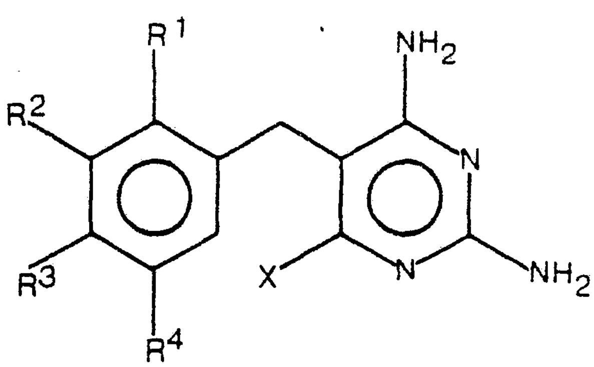

III

wherein
$R^1$ represents hydrogen or $C_{1-6}$-alkyl; $R^2$ represents hydrogen and $R^3$ and $R^4$ represent $C_{1-6}$-alkylthio or $R^1$ represents hydrogen; $R^2$ and $R^4$ represent $C_{1-6}$-alkylthio and $R^3$ represents $C_{1-6}$-alkoxy, $C_{1-6}$-alkylthio or pyrrolo or $R^1$ represents hydrogen; $R^2$ represents $C_{1-6}$-alkoxy and $R^3$ and $R^4$ represent $C_{1-6}$-alkylthio or $R^1$ represents hydrogen; $R^2$ and $R^4$ represent $C_{1-6}$-alkoxy and $R^3$ represents $C_{1-6}$-alkylthio, carboxy-$C_{1-6}$-alkylthio or cyano-$C_{1-6}$-alkylthio and X is halogen.

2. Benzylpyrimidines of the general formula

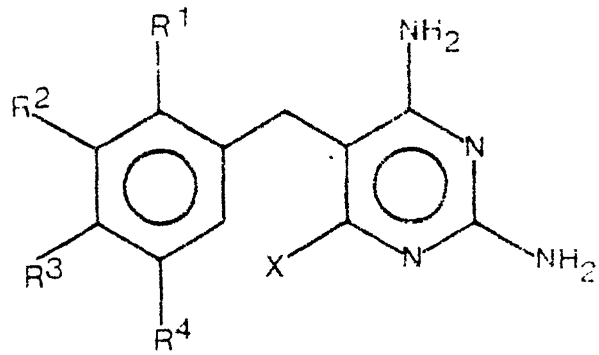

II

wherein
$R^1$ represents hydrogen or $C_{1-6}$-alkyl; $R^2$ represents hydrogen and $R^3$ and $R^4$ represent $C_{1-6}$-alkylthio or $R^1$ represents hydrogen; $R^2$ and $R^4$ represent $C_{1-6}$-alkylthio and $R^3$ represents $C_{1-6}$-alkoxy, $C_{1-6}$-alkylthio or pyrrolo or $R^1$ represents hydrogen; $R^2$ represents $C_{1-6}$-alkoxy and $R^3$ and $R^4$ represent $C_{1-6}$-alkylthio or $R^1$ represents hydrogen; $R^2$ and $R^4$ represent $C_{1-6}$-alkoxy and $R^3$ represents $C_{1-6}$-alkylthio or carboxy-$C_{1-6}$-alkylthio and X is halogen.

3. 2,6-Diamino-4-chloro-5-(3,5-dimethoxy-4-methylthiobenzyl)-pyrimidine.

## Revendications

1. Benzylpyrimidines de formule générale

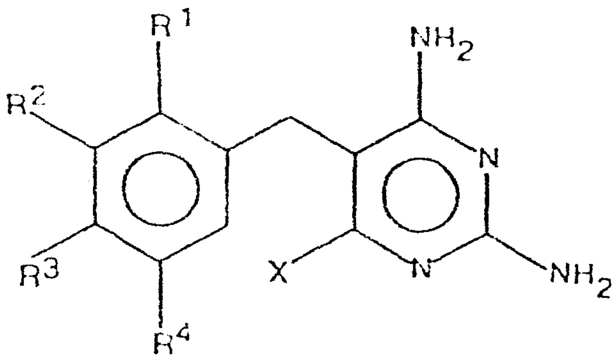

II

où
$R^1$ représente un hydrogène ou un alcoyle en $C_1$ à $C_6$; $R^2$ représente un hydrogène et $R^3$ et $R^4$ un alcoylthio en $C_1$ à $C_6$ ou $R^1$ représente un hydrogène; $R^2$ et $R^4$ un alcoylthio en $C_1$ à $C_6$ et $R^3$ un alcoxy en $C_1$ à $C_6$, un alcoylthio en $C_1$ à $C_6$ ou un pyrrolo ou $R^1$ représente un hydrogène; $R^2$ un alcoxy en $C_1$ à $C_6$ et $R^3$ et $R^4$ un alcoylthio en $C_1$ à $C_6$ ou $R^1$ représente un hydrogène; $R^2$ et $R^4$ un alcoxy en $C_1$ à $C_6$ et $R^3$ un alcoylthio en $C_1$ à $C_6$, un carboxy-alcoylthio en $C_1$ à $C_6$ ou un cyano-alcoylthio en $C_1$ à $C_6$ et X est un halogène.

2. Benzylpyrimidines de formule générale

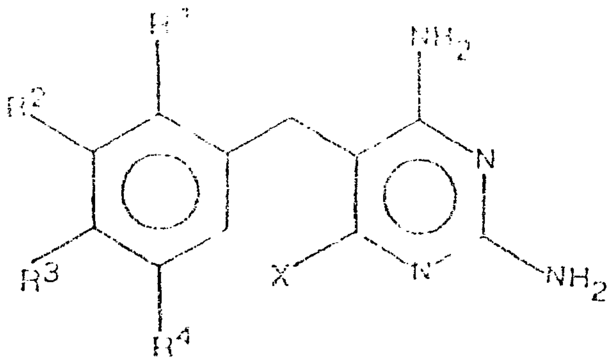

III

où

$R^1$ représente un hydrogène ou un alcoyle en $C_1$ à $C_6$; $R^2$ un hydrogène et $R^3$ et $R^4$ un alcoylthio en $C_1$ à $C_6$ ou

$R^1$ représente un hydrogène; $R^2$ et $R^4$ un alcoylthio en $C_1$ à $C_6$ et $R^3$ un alcoxy en $C_1$ à $C_6$, un alcoylthio en $C_1$ à $C_6$ ou un pyrrolo ou

$R^1$ représente un hydrogène; $R^2$ un alcoxy en $C_1$ à $C_6$ et $R^3$ et $R^4$ un alcoylthio en $C_1$ à $C_6$ ou

$R^1$ représente un hydrogène; $R^2$ et $R^4$ un alcoxy en $C_1$ à $C_6$ et $R^3$ un alcoylthio en $C_1$ à $C_6$ ou un carboxy-($C_1-C_6$)-alcoylthio et

X est un halogène.

3. 2,6-diamino-4-chloro-5-(3,5-diméthoxy-4-méthylthiobenzyl)-pyrimidine.